# EUROPEAN PATENT APPLICATION

(11) **EP 1 447 108 A1**
(43) Date of publication of application: **18.08.2004**
(21) Application number: 03252192.4
(22) Date of filing: 07.04.2003
(51) Int. Cl.: A61M 5/32

(54) **A safety accessory for needles**

(30) Priority: 11.02.2003 GB 0303026
(71) Applicant: Weston, Terence Edward, Swannington, Norfolk NR9 5NP (GB); Emmott, Douglas Arthur, Swilland, Ipswich, Suffolk IP6 9LR (GB)
(72) Inventor: Weston, Terence Edward, Swannington, Norfolk NR9 5NP (GB); Emmott, Douglas Arthur, Swilland, Ipswich, Suffolk IP6 9LR (GB)
(74) Representative: Gillard, Richard Edward

(57) **Abstract**

This invention relates to an accessory for attaching to a needle (3) to prevent needle stick injuries. The safety needle accessory is suitable for use with a hollow needle (3) and comprises a needle hub (7) capble of surrounding the needle (3) and a slidable sleeve (5) slidably mounted on the needle hub (7). The slidable sleeve (5) bears resiliently on the needle hub (7) such that, in use, as the slidable sleeve (5) is caused to move into a retracted position. A restoring force is generated within the slidable sleeve (5) and on removal of the needle (3) from the patient the restoring force causes the slidable sleeve (5) to move into an extended position covering the needle tip. The slidable sleeve (5) locks in this position after removal of the needle from the patient.

## Description

This invention relates to a safety needle and in particular to an accessory for attaching to a needle.

Needle stick injuries carry a significant risk of spreading infection, such as HIV and hepatitis, and are commonplace among healthcare workers. The USA has led the way in introducing legislation that obliges healthcare providers to use the safest devices when giving injections, intravenous drug administration and similar invasive procedures. Other countries are following and, even without legislation, the ever-present risk of litigation has alerted pharmaceutical companies and health authorities to seek suitable safe devices.

As a result of the heightened awareness of needle stick injuries, there have been a large number of inventions purporting to address this issue. Most take the form of a protective sleeve which covers the needle tip after the injection has been given, or means for retracting the needle rapidly into the syringe barrel. In the former case, a weakness of the designs has been the need for the user to perform an action to render the needle safe; thus if the step is omitted, the risk remains. Such a safety needle is exemplified by US 4,911,693. In the latter case, the needle retraction mechanism requires that the syringe plunger is pushed to the end of its stroke in order to activate the retraction mechanism. In other words, virtually none of the devices are "fail safe". In many real-life situations, the patient can involuntarily react to the pain of the injection and pull away from the needle, exposing the sharp tip, and therefore presenting a risk of a needle stick.

Another drawback of prior art safety needles (which in the present context includes safety syringes) is that they are not compatible with current accepted practice. The problems includes drug incompatibility with the device construction materials, difficulty in using standard sterilising methods, difficulty in fitting to the syringe, large size, difficulty in filling, and very high cost.

Again, a common requirement for drug administration is to draw the drug from a vial or bulk container, or first reconstitute a lyophilised drug, and then draw up the required volume of drug from the reconstitution vessel.. Good practice demands that separate needles are used for such procedures, and from a practical viewpoint, the needle used to draw up the drug usually will be larger than the delivery needle. Most of the prior art devices do not address this aspect.

Another common requirement is for pre-filled syringes, and for reasons of drug compatibility and long-term storage, the syringe barrel is often made from glass, with the hypodermic needle bonded into the delivery end of the syringe barrel. Alternatively, a few drugs are compatible with plastics, and there are available plastic syringe barrels with moulded or bonded-in hypodermic needles.

Some safety devices have addressed the problems associated with manually operated safety sleeves, such as the devices disclosed in US 4,813,940 and US 5,104,384. High cost has, however, tended to be a significant deterrent which has resulted in poor commercial success of the better devices. Potential specifiers and purchasers are put off by the high cost (several times that of a basic syringe), and have been able to cite the fallibility of the devices which are available as a way of avoiding buying them.

Again, there is a huge "inertia" in the world's healthcare system. The massive investment in manufacturing plants, aseptic production and assembly, sterilisation, existing long-term supply contracts, the bewildering number of so-called safety syringes on offer, and the drawbacks already noted, have mitigated against rapid adoption of safety needles and syringes.

There is therefore still a need for a low cost safety needle which prevents needle stick injuries in healthcare workers.

Accordingly, the present invention provides a safety needle accessory suitable for use with a hollow needle comprising a needle hub capable of surrounding the needle and a slidable sleeve slidably mounted on the needle hub, the needle hub and the slidable sleeve having a receiving end which, in use, is distal to the tip of the needle and an injection end which is proximal to the tip of the needle, wherein the receiving end of the needle hub is suitable for connection to an injection device and the slidable sleeve is adapted to slide in the direction of the length of the needle between an extended position in which the tip of the needle is located inside the slidable sleeve and a retracted position in which the tip of the needle projects from the slidable sleeve such that, in use, as the needle is inserted into a patient, the slidable sleeve is caused to move into the retracted position, and wherein the needle hub has an outer surface which is adapted to deflect the slidable sleeve as, in use, the needle is inserted into the patient, and the slidable sleeve bears resiliently on the outer surface of the needle hub such that, in use, as the slidable sleeve is caused to move towards the receiving end of the needle hub and into the retracted position, a restoring force is generated within the slidable sleeve and on removal of the needle from the patient the restoring force causes the slidable sleeve to move towards the injection end of the needle hub and into the extended position, the safety needle further comprising a locking mechanism capable of retaining the slidable sleeve in an extended position after removal of the needle from the patient.

This safety needle accessory has a low manufacturing cost, suits all types and sizes of injection devices, e.g. syringes, is automatically safe in any circumstance, and allows needles to be changed safely when drawing from vials and administering reconstituted drugs.

The present invention also provides a safety needle, an injection device and a syringe incorporating the safety needle accessory as defined above. The safety needle comprises a hollow needle and the safety needle accessory as defined above, and the syringe comprises a piston, a barrel and the safety needle as defined above

The present invention will now be described with reference to the following drawings, in which:
Fig. 1 shows a safety needle accessory of the present invention attached to a syringe;
Fig. 2 shows a centre line cross-section through the safety needle accessory of the present invention attached to a syringe;
Fig. 3 shows the slidable sleeve partly retracted to expose the hollow needle;
Fig. 4 shows the safety needle accessory with the slidable sleeve in an extended and locked position;
Fig. 5 shows the detent mechanism;
Fig. 6 shows the receiving end of the slidable sleeve showing the cantilever arms;
Fig. 7 shows the barrel of a typical glass syringe used commonly for pre-filling with a drug, assembled with the safety needle accessory of the present invention; and
Fig. 8 shows an integral syringe barrel and needle hub in accordance with the present invention.

In the drawings, like parts are given the same reference numerals.

The standard construction of a needle comprises a plastic needle hub which is tightly formed onto the needle (by moulding, gluing or thermal upsetting). The needle is protected by a plastic slidable sleeve which fits firmly but removably onto the needle hub. Normally the needle is packed in a pouch and the whole is sterilised, usually by gamma irradiation. Thus the typical needle comprises three parts, namely a hollow needle, a needle hub, and a removable sleeve.

The present invention may be conceptualised by comparing it with a conventional hypodermic needle: the component replacing the normal needle hub is one half of a returning mechanism, and the part that would be the protective slidable sleeve forms the other half of the mechanism, whilst also protecting the needle. Together with the needle, the component count is three, exactly the same as for a conventional hypodermic needle, and therefore a similar low manufacturing cost. Furthermore, the user does not have to learn a new technique, and so the adoption of the safety needle will not require special training of healthcare workers.

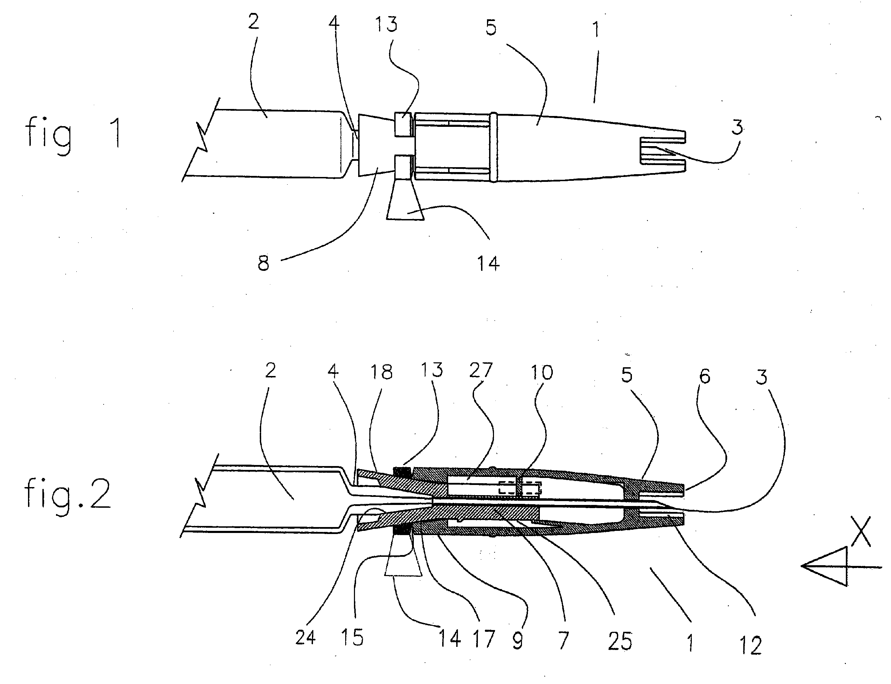

Fig. 1 shows the general arrangement of the safety needle 1 of the present invention as fitted to a male Luer taper connector 4 of syringe 2, with the end of the hollow (e.g. hypodermic) needle 3 enclosed by the slidable sleeve 5. The slidable sleeve 5 is prevented from longitudinal movement on the needle hub 8 by a locking ring 13, which may be removed by pulling on the tab 14. The removable locking ring may be connected to the needle hub and/or the slidable sleeve. Although embodied in the Figs. as cylindrical, the cylindrical shape of the needle hub and slidable sleeve may be replaced by triangular, rectangular or other shapes to suit the application.

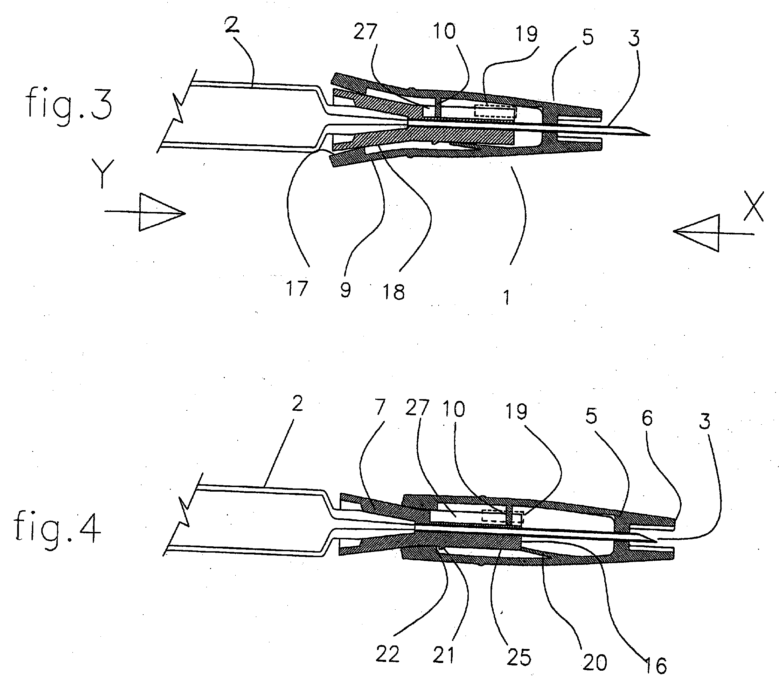

Fig. 2 is a cross-section through the axis of the safety needle 1. The needle hub 7 is cylindrical and terminates at the end which receives the syringe with a conical section 8, and is moulded onto the needle 3. The conical section 8 has an inner female Luer cone 24 which is shown frictionally attached to the male Luer cone 4 of syringe 2 (the Luer system for attaching the needle to the syringe has two main forms, that is a taper friction fit and a screw thread and both are included in the present invention). The cylindrical slidable sleeve 5 shrouds the needle 3 and the needle hub 7, and is freely sliding on and guided by the needle hub 7. At the receiving end (i.e. the syringe end) of slidable sleeve 5, there are four cantilever arms 9 which bear resiliently upon the surface 18 of the conical section 8. The slidable sleeve 5 is free to slide on the needle hub 7, but is temporarily prevented from doing so by the locking ring 13. Locking ring 13 is moulded integrally with the slidable sleeve 5 by a frangible joint 15, and may be partially or wholly detached by pulling on the tab 14 to break the frangible joint 15. It is preferred that the ring 13 remains attached to the slidable sleeve 5 to reduce the number of discarded parts. In addition, the frangible joint provides a tamper-evident lock. Alternatively, the locking ring 13 may be moulded to the needle hub 8 via a suitable frangible connection. When the locking ring 15 is removed, as shown in Fig. 3 the slidable sleeve 5 may be pushed in the direction of arrow X by acting on the face 6, when it will move relative to the needle hubs 7, 8 to expose the needle 3. As the slidable sleeve 5 moves, the cantilever arms 9 are forced outwards by the surface 18 of the conical section 8. The cantilever arms 9 are resilient and the reaction force against the surface 18 produce a resultant force Y acting against arrow X, so that when the original force is removed, the slidable sleeve 5 returns to cover the tip of needle 3.

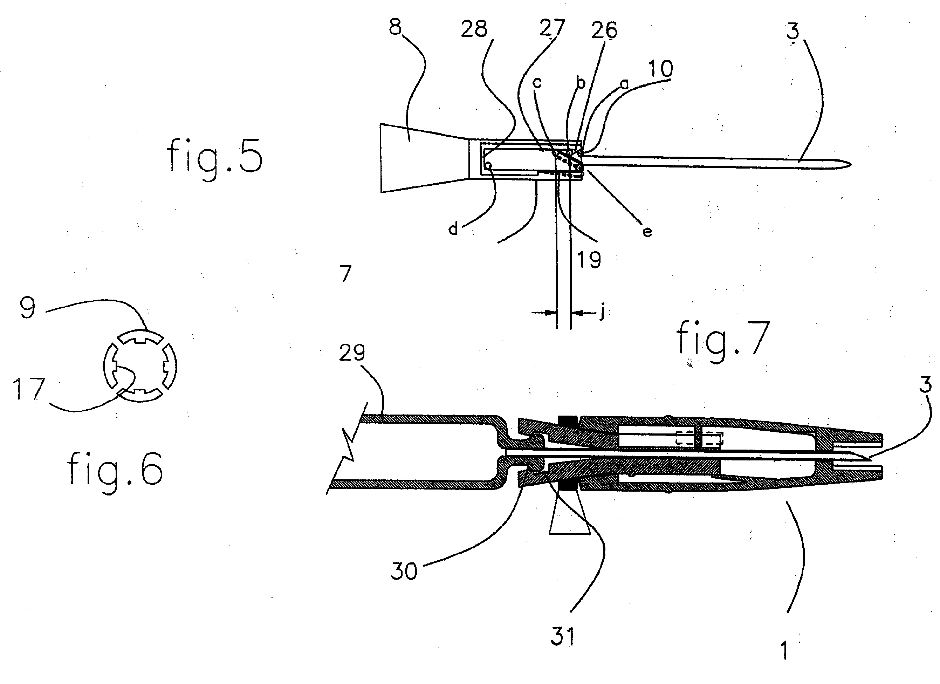

As shown in Fig. 6, the cantilever arms 9 may have pads 17 which bear onto the surface 18 and, by suitably designing the bearing surfaces of the pads 17, various spring characteristics may be obtained. Although four cantilever arms 9 are shown, any number could be employed. At least one cantilever arm 9 is required for this embodiment although 2 to 6 are preferred and particularly preferably four.

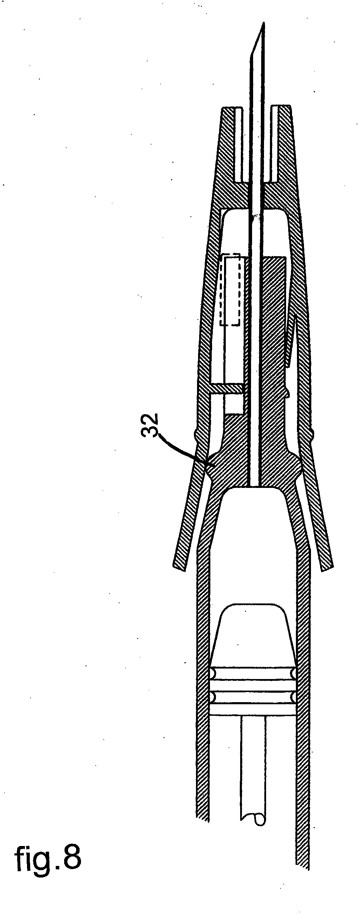

Although the surface 18 is exemplified by a conical surface, other embodiments may be used within the scope of the present invention. In the Figs the surface is straight, i.e. substantially conical by which the applicant means sufficiently conical to generate a restoring force, however, the surface need not be straight as shown, but may be curved to give a more linear return rate. Thus the force Y could be substantially constant over a reasonable working stroke of the slidable sleeve 5. In addition, the whole surface 18 of the receiving end of the needle hub 17 need not be conical. In fact just one tapered section, e.g.. a tapered ridge, would be sufficient. The tapered section does not have to project from the surface of the needle hub 7. The tapered section could also descend into the wall of the needle hub, i.e. a tapered detent rather than a tapered ridge. Also, as described below with reference to Fig. 8, provided the slidable sleeve 5 is suitably configured, a projection in the surface 18 of the needle hub 7 will suffice.

These different arrangements provide a great deal of design flexibility in the safety needle accessory of the present invention. For example, the linearity of the return rate may be varied depending on the particular requirements for a particular application.

Referring to Fig. 4, when the slidable sleeve 5 returns in direction Y, it travels slightly past its original staring position, so that the resilient pawl 20 which was depressed by the surface 25 of hub 7, snaps out to act against face 16 of hub 7. This ensures that the slidable sleeve 5 cannot be pushed back towards the syringe 2, and therefore the needle 3 is safely covered. It is preferred that there is a pre-load between the cantilever arms 9 and hub 8 to ensure that the slidable sleeve 5 is sufficiently biased in the direction of arrow Y to complete its full displacement potential.

The slidable sleeve preferably has a first extended position where the slidable sleeve is able to be moved towards the receiving end of the needle hub and a second extended position where the slidable sleeve is in a locked position. The different start and finish positions of the slidable sleeve 5 is achieved by a detent mechanism shown in Fig. 5, which is to be read in conjunction with Figs. 2, 3 and 4. After the first two or three millimetres of movement, a detent integral with the slidable sleeve "switches" so that the return of the slidable sleeve trips a latching pawl, so that the slidable sleeve returns only to the "safe" position; that is the pawl prevents the slidable sleeve from being moved towards the syringe a second time, and thus protects the tip of the needle. As part of the detent mechanism, an inside surface of the slidable sleeve has a pin 10 which engages a sprag 26 and a resilient pawl 19 attached to the needle hub 7 thereby holding the slidable sleeve in the first extended position and, in use, allowing the slidable sleeve to move into the second extended position. Pin 10 is integral or attached to the slidable sleeve 5. Sprag 26, resilient pawl 19, and recess 27 are moulded integrally with the needle hubs 7, 8, and the pin 10 extends into the recess, and allowed to move freely except where controlled by the detent and the boundaries of the recess. In the initial assembly of the safety needle, the slidable sleeve 5 is placed over the needle 3 with pin 10 proximate to the sprag 26 (formed as part of the needle hub 7) at position a (Fig. 5). As the slidable sleeve 5 is moved further, the pin 5 deflects the resilient pawl 19, until the pin 10 is trapped behind sprag 26 at position b. In this position the slidable sleeve 5 is trapped on the needle hubs 7, 8 and cannot be removed without applying considerable force. This is the position of the components as supplied to the end user, and the location of the locking ring 13 takes account of this. With the locking ring 13 removed, the slidable sleeve 5 is pushed further towards the syringe 2, and pin 10 again deflects the resilient pawl 19 until the pin 10 reaches position c. This distance j defines the initial displacement of the slidable sleeve 5, when starting the injection, and the tip of needle 3 may be level with the face 6 of slidable sleeve 5. The slidable sleeve 5 may now be moved towards the syringe until the pin 10 reaches the end wall 28 of the recess 27 at position d. This position defines the maximum displacement of slidable sleeve 5, and thus the maximum exposure of the needle 3. At any time the force acting on slidable sleeve 5 is removed, the slidable sleeve 5 will return in the direction of arrow Y until pin 10 reaches the position e. Pin 10 would also help prevent the removal of slidable sleeve 5, but additionally the tooth 21 is now proximate to face 22 on a cantilever arm 9, which prevents the removal of the slidable sleeve 5. In this final position, pawl 20 engages with face 16 of hub 7 and prevents the slidable sleeve 5 from being moved. It should be noted that with the present diagrammatic presentation of the safety needle, a small amount of rotational movement is necessary between the slidable sleeve 5 and hubs 7,8 to permit the pin 10 to move from position c to position e, but the rotation is preferably negligible.

The detent mechanism is interchangeable between the slidable sleeve 5 and needle hub 7 if required. Also, the detent mechanism described hereinabove is but one of a number of such mechanisms, the main requirement being to permit the following sequence of operation: permit the slidable sleeve to be moved sufficiently so that the opening in the slidable sleeve is level with or just in front of the needle tip, at which position the detent must be activated so that if the displacing force on the slidable sleeve is removed, the slidable sleeve slides forward and locks, thus protecting the user from contact with the needle tip. Typically, the tip of the needle would be about 3 mm back from the face of the opening in the slidable sleeve at the start, and 1 mm back from the face when the detent is activated.

As an alternative to being supplied pre-assembled, the safety needle accessory may be supplied without a needle and then the accessory is attached to an injection device having an integral needle. Fig. 7 shows the device 1 as previously described, except that in this embodiment the needle 3 is bonded into the outlet end of the syringe barrel 29. The needle is free to pass through the needle hub 7, and the cone 9 is adapted at 31 to snap-fit over the projection 30. The form of projection 30 is produced as a result of rolling the glass onto a mandrel, whereby the excess glass is forced in to the shape as shown. Alternatively, a more defined snap-fitting termination may be formed, the object being to make the device 1 difficult to remove after assembling it to the syringe barrel.

Fig. 8 shows a safety needle 1 in which the needle hub 7 is integral with the barrel of the syringe 2. The slidable sleeve 5 is then attached to the needle hub 7 in the manner as described hereinbelow. The material of the needle hub 7, which also constitutes the barrel of the syringe, would, of course, have to be made from a drug-compatible material.

As mentioned hereinabove, Fig. 8 also shows the separate feature of the projection 32 on the surface 18 of the needle hub 7 which may be used to deflect the slidable sleeve 5 thereby generating the restoring force. This embodiment, i.e. incorporating the projection 32, provides a highly non-linear return rate since the length of the slidable sleeve 5 is effectively reduced as the slidable sleeve 5 and the needle hub 7 are slid together, thereby increasing the stiffness of the slidable sleeve 5.

As an alternative to the tapered outer surface of the needle hub 7, the slidable sleeve has at least one cantilever arm which engages a helical track in the outer surface of the needle hub such that, in use, as the needle is inserted into a patient, the at least one cantilever arm is displaced radially by the helical track in the outer surface of the needle hub thereby generating the restoring force. Thus, as the slidable sleeve 5 is caused to move towards the receiving end of the needle hub 7, one or more cantilever arms 9 are forced to follow the direction of the helical tracks. Since the cantilever arms 9 are resilient, a restoring force will be generated.

As an alternative to cantilever arms, the receiving end of the slidable sleeve itself may have elastic properties such that, in use, as the needle is inserted into a patient, the restoring force is generated within the slidable sleeve. By elastic properties the applicant means that the restoring force is generated within a radially continuous slidable sleeve, i.e. a sleeve without cantilever arms. The elastic properties may be achieved by using an elastic material, such as an elastomeric polymer. Alternatively, the receiving end of the slidable sleeve may be concertinaed, with the ridges, of course, running parallel to the hollow needle. The elastic properties could also be generated using a circumambient spring attached to the slidable sleeve.

Importantly, the cost of the components of the present invention as well as the assembly costs will be of the same order as those for a conventional needle.

It is preferable that the coefficient of friction between the slidable sleeve 5 and the needle hub 7 is low, so that the resultant biasing force to return the slidable sleeve is not compromised by "stiction", or so high that the force required on the patient's skin to deflect the slidable sleeve 5 is excessive. This may be achieved by careful selection of materials. Such materials are known in the art, for example, the needle hub could be made from a high-density polyethylene or similar drug-compatible plastics material, and the slidable sleeve from polycarbonate or polyester. If necessary, a lubricant may be used, or a lubricant may be incorporated with the polymers. Generally the materials should be suitable for sterilisation by gamma radiation, but it is possible to select materials compatible with sterilisation by steam or other gas such as ethylene oxide.

The safety needle 1 may be supplied pre-assembled as shown in Figs. 1 and 2, and attached by the user to a previously filled syringe 2 by pushing the female Luer 24 of the needle hub 8 onto the male Luer connector 4. The locking ring 13 prevents any displacement of slidable sleeve 5 relative to the needle hub 8. The user releases the locking ring, and may give an injection to the patient by placing the face 6 onto the injection site and pushing on the syringe body in the direction of arrow Y in the normal way of giving an injection. This causes the needle 3 to penetrate the patient's tissue as the slidable sleeve 5 is displaced by the reaction against the patient's skin. The syringe plunger is depressed to dispense the medicament, and the syringe is withdrawn from the patient. The cantilever springs 9 urge the slidable sleeve 5 towards the tip of the needle 3 in the direction of arrow Y, so that the tip of needle 3 is immediately covered. The pawl 20 will have engaged automatically with the face 16 of hub 7 to prevent the slidable sleeve 5 from being pushed back again. The syringe and needle may be disposed of safely without risk of needle stick injury.

Thus the procedure for using the safety needle is the same as with using conventional needles. The act of releasing the locking ring is similar to removing the temporarily captive needle cover from a conventional needle.

As previously mentioned hereinabove, the syringe may be supplied emply or pre-filled. When a pre-filled syringe is used, the syringe is preferably sealed using a sealing cap or plug to prevent evaporation or loss of the drug, excipient, carrier and/or diluent by, for example, thermal expansion.

As well as application to a syringe, the same safety needle accessory described herein could form the basis of a intravenous giving set, so that the insertion of the needle into the patient's vein is simple and safe. Indeed, the safety needle accessory of the present invention may be used with any suitable injection device.

## Claims

1. A safety needle accessory suitable for use with a hollow needle comprising
a needle hub capable of surrounding the needle and
a slidable sleeve slidably mounted on the needle hub,
the needle hub and the slidable sleeve having a receiving end which, in use, is distal to the tip of the needle and an injection end which is proximal to the tip of the needle,
wherein the receiving end of the needle hub is suitable for connection to an injection device and the slidable sleeve is adapted to slide in the direction of the length of the needle between an extended position in which the tip of the needle is located inside the slidable sleeve and a retracted position in which the tip of the needle projects from the slidable sleeve such that, in use, as the needle is inserted into a patient, the slidable sleeve is caused to move into the retracted position,
and wherein the needle hub has an outer surface which is adapted to deflect the slidable sleeve as, in use, the needle is inserted into the patient, and the slidable sleeve bears resiliently on the outer surface of the needle hub such that, in use, as the slidable sleeve is caused to move towards the receiving end of the needle hub and into the retracted position, a restoring force is generated within the slidable sleeve and on removal of the needle from the patient the restoring force causes the slidable sleeve to move towards the injection end of the needle hub and into the extended position,
the safety needle further comprising a locking mechanism capable of retaining the slidable sleeve in an extended position after removal of the needle from the patient.

2. A safety needle accessory as claimed in claim 1, wherein at least part of the receiving end of the needle hub has a tapered outer surface which tapers towards the injection end such that, in use, as the needle is inserted into a patient, the slidable sleeve is displaced outwards by the tapered outer surface of the needle hub thereby generating the restoring force.

3. A safety needle accessory as claimed in claim 2, wherein the outer surface of the receiving end of the needle hub has a substantially conical shape which tapers towards the injection end.

4. A safety needle accessory as claimed in any preceding claim, wherein the slidable sleeve has at least one cantilever arm which bears resiliently on the tapered outer surface of the needle hub and, in use, as the needle is inserted into a patient, the at least one cantilever arm is displaced outwards by the tapered outer surface of the needle hub thereby generating the restoring force.

5. A safety needle accessory as claimed in any preceding claim, wherein the receiving end of the slidable sleeve has elastic properties and, in use, as the needle is inserted into a patient, the restoring force is generated within the slidable sleeve.

6. A safety needle accessory as claimed in claim 5, wherein the receiving end of the slidable sleeve is concertinaed.

7. A safety needle accessory as claimed in any of claims 1 to 4, wherein the slidable sleeve has at least one cantilever arm which engages a helical track in the outer surface of the needle such that, in use, as the needle is inserted into a patient, the at least one cantilever arm is displaced radially by the helical track in the outer surface of the needle hub thereby generating the restoring force.

8. A safety needle accessory as claimed in any preceding claim, wherein the slidable sleeve is pre-loaded with a restoring force when the slidable sleeve is in the extended position.

9. A safety needle accessory as claimed in claims 4 or 7, wherein the safety needle has 2 to 6 cantilever arms.

10. A safety needle accessory as claimed in claim 9, wherein the safety needle has 4 cantilever arms.

11. A safety needle accessory as claimed in any preceding claim, wherein the accessory further comprising a removable locking ring connected to the needle hub and/or the slidable sleeve which, when connected, is capable of preventing movement of the sleeve.

12. A safety needle accessory as claimed in claim 11, wherein the removable locking ring is frangibly connected to the needle hub and/or the slidable sleeve

13. A safety needle accessory as claimed in any preceding claim, wherein the slidable sleeve has a first extended position where the slidable sleeve is able to be moved towards the receiving end of the needle hub and a second extended position where the slidable sleeve is in a locked position.

14. A safety needle accessory as claimed in claim 13, wherein the locking mechanism comprises a latching pawl on the inside surface of the slidable sleeve capable of latching to a shoulder on the outside surface of the needle hub when the slidable sleeve is in the second extended position.

15. A safety needle accessory as claimed in claim 14, wherein an inside surface of the slidable sleeve has a pin which engages a sprag and a resilient pawl attached to the needle hub thereby holding the slidable sleeve in the first extended position and, in use, allowing the slidable sleeve to move into the second extended position.

16. A safety needle accessory as claimed in any preceding claim, wherein the accessory is constructed from materials capable of being sterilised with gamma irradiation.

17. A safety needle comprising a hollow needle and the safety needle accessory as claimed in any preceding claim.

18. A safety needle as claimed in claim 17, wherein the hollow needle is integral with the safety needle accessory.

19. A safety needle as claimed in claims 17 or 18, wherein the receiving end of the needle hub is adapted for connection to a Luer connector.

20. An injection device comprising the safety needle as claimed in any of claims 17 to 19.

21. A syringe comprising a piston, a barrel and the safety needle as claimed in any of claims 17 to 19.

22. A syringe as claimed in claim 21 wherein the needle hub is integral with the barrel.
